(19) Europäisches Patentamt
European Patent Office
Office européen des brevets

(11) **EP 4 382 594 A1**

(12) **EUROPEAN PATENT APPLICATION**
published in accordance with Art. 153(4) EPC

(43) Date of publication:
**12.06.2024 Bulletin 2024/24**

(21) Application number: **22852908.7**

(22) Date of filing: **26.07.2022**

(51) International Patent Classification (IPC):
*C12M 1/00* (2006.01)        *C12M 3/00* (2006.01)

(52) Cooperative Patent Classification (CPC):
**C12M 1/00; C12M 3/00**

(86) International application number:
**PCT/JP2022/028831**

(87) International publication number:
**WO 2023/013485 (09.02.2023 Gazette 2023/06)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **04.08.2021 JP 2021128434**

(71) Applicant: **Nikkiso Co., Ltd.
Tokyo 150-6022 (JP)**

(72) Inventors:
• **KITAGAWA, Fumihiko**
  **Kanazawa-shi Ishikawa 920-0177 (JP)**
• **JIMBO, Yoichi**
  **Kanazawa-shi Ishikawa 920-0177 (JP)**
• **WATANABE, Yosuke**
  **Kanazawa-shi Ishikawa 920-0177 (JP)**

(74) Representative: **Algemeen Octrooi- en
Merkenbureau B.V.
P.O. Box 645
5600 AP Eindhoven (NL)**

(54) **CULTURE DEVICE AND CULTURE METHOD**

(57)   A culture device (1) includes: a culture vessel (2) that contains an aggregate (S) of cells and a culture medium (W); a suction tube (4) that suctions the culture medium (W) in the culture vessel (2); a suction unit (8) that generates suction force in the suction tube (4); and a control unit (14) that controls the suction unit (8) based on an antagonistic suction flow velocity of the aggregate (S), which is a velocity at which sedimentation of the aggregate (S) is antagonistic to floating of the aggregate (S) due to the suction of the culture medium (W).

FIG. 1

**Description**

TECHNICAL FIELD

The present invention relates to a culture device and a culture method.

BACKGROUND ART

Conventionally, as a method for mass culturing cells, suspension stirred culture of cell aggregates with use of a culture tank has been known. With regard to such a culture method, Patent Literature 1 discloses a cell culturing apparatus equipped with an aspirator for suctioning a culture medium. By suctioning the culture medium with the aspirator to replace the culture medium, for example, the efficiency of cell culture can be improved.

The aspirator of Patent Literature 1 has a double-tube structure including an outer tube and an inner tube. The outer tube includes a filter through which the culture medium passes, and the inner tube suctions the culture medium that has passed through the filter. The outer tube also has an air hole that connects the inner space of the outer tube and the outside, so that excessive negative pressure in the outer tube can be released to the outside. In Patent Literature 1, such a structure prevents the filter from being clogged with cell aggregates having sizes equal to or larger than the mesh size.

PRIOR ART REFERENCE

PATENT LITERTURE

Patent Literature 1: WO 2019/124540

SUMMARY OF INVENTION

TECHNICAL PROBLEM

Even if excessive negative pressure is made not to occur in the outer tube, as long as the filter is provided in the outer tube, it is inevitable that aggregates will come into contact with the filter. As long as there is a possibility that aggregates come into contact with the filter, there is a risk that the aggregates may adhere to the filter. If aggregates adhere to the filter, it may inhibit the suction of the culture medium, so that the efficiency of the cell culture may be reduced. In addition, the growth and proliferation of cells may also be inhibited, and the efficiency of the cell culture may be reduced also in this respect.

The present invention has been made in view of such a situation, and a purpose thereof is to provide a technology for improving the efficiency of cell culture.

SOLUTION TO PROBLEM

A culture device according to one embodiment of the present invention includes: a culture vessel that contains one or more aggregates of cells and a culture medium; a suction tube that suctions a culture medium in the culture vessel; a suction unit that generates suction force in the suction tube; and a control unit that controls the suction unit based on an antagonistic suction flow velocity of an aggregate, which is a velocity at which sedimentation of the aggregate is antagonistic to floating of the aggregate due to the suction of the culture medium.

A culture method according to another embodiment of the present invention includes suctioning, from a culture vessel that contains one or more aggregates of cells and a culture medium, the culture medium at a flow velocity corresponding to an antagonistic suction flow velocity of an aggregate, which is a velocity at which sedimentation of the aggregate is antagonistic to floating of the aggregate due to the suction of the culture medium.

Optional combinations of the aforementioned constituting elements, and implementation of the present invention, including the constituting elements and expressions, in the form of methods, apparatuses, or systems may also be practiced as additional modes of the present invention.

ADVANTAGEOUS EFFECTS OF INVENTION

According to the present invention, the efficiency of cell culture can be improved.

BRIEF DESCRIPTION OF DRAWINGS

[FIG. 1] FIG. 1 is a schematic diagram of a culture device according to a first embodiment.
[FIG. 2] FIG. 2 is a diagram that shows relationships between the aggregate particle size and the sedimentation velocity.
[FIG. 3] FIG. 3 is a diagram that shows states of aggregates when the culture medium is suctioned at various suction flow velocities.
[FIG. 4] FIG. 4 is a diagram that shows relationships between the sedimentation velocities and the antagonistic suction flow velocities of aggregates.
[FIG. 5] FIG. 5 is a diagram that shows relationships between $1/W_*$ and $1/S_*$.
[FIG. 6] FIG. 6 is a diagram that shows a sedimentation velocity prediction curve.
[FIG. 7] FIG. 7 is a diagram that shows an antagonistic suction flow velocity prediction curve.
[FIG. 8] FIG. 8 is a diagram that shows a predicted value of the sedimentation velocity, a predicted value of the antagonistic suction flow velocity, and an antagonistic flow rate at each tube diameter, for each aggregate particle size.
[FIG. 9] FIG. 9 is a schematic diagram of a culture device according to a second embodiment.
[FIG. 10] FIG. 10 is a schematic diagram of a culture device according to a third embodiment.
[FIG. 11] FIG. 11 is a schematic diagram of a culture device according to a fourth embodiment.
[FIGS. 12] FIG. 12A is an optical microscope image of a culture medium in Comparative Example 1. FIG. 12B is an optical microscope image of a filter in Comparative Example 1. FIG. 12C is an optical microscope image of a culture medium in Example 1.
[FIG. 13] FIG. 13 is a diagram that shows an aggregate particle size distribution.
[FIG. 14] FIG. 14 is an optical microscope image of aggregates deposited in a bottom part of a recovery suction tube.

DESCRIPTION OF EMBODIMENTS

In the following, the present invention will be described based on preferred embodiments with reference to the drawings. The embodiments are intended to be illustrative only and not to limit the invention, so that it should be understood that not all of the features or combinations thereof described in the embodiments are necessarily essential to the invention. Like reference characters denote like or corresponding constituting elements, members, and processes in each drawing, and repetitive description will be omitted as appropriate. Also, the scale or shape of each component shown in each drawing is defined for the sake of convenience to facilitate the explanation and is not to be regarded as limitative unless otherwise specified. Also, when the terms "first", "second", and the like are used in the present specification or claims, such terms do not imply any order or degree of importance and are used to distinguish one configuration from another. Further, in each drawing, part of a member less important in describing embodiments may be omitted.

First Embodiment

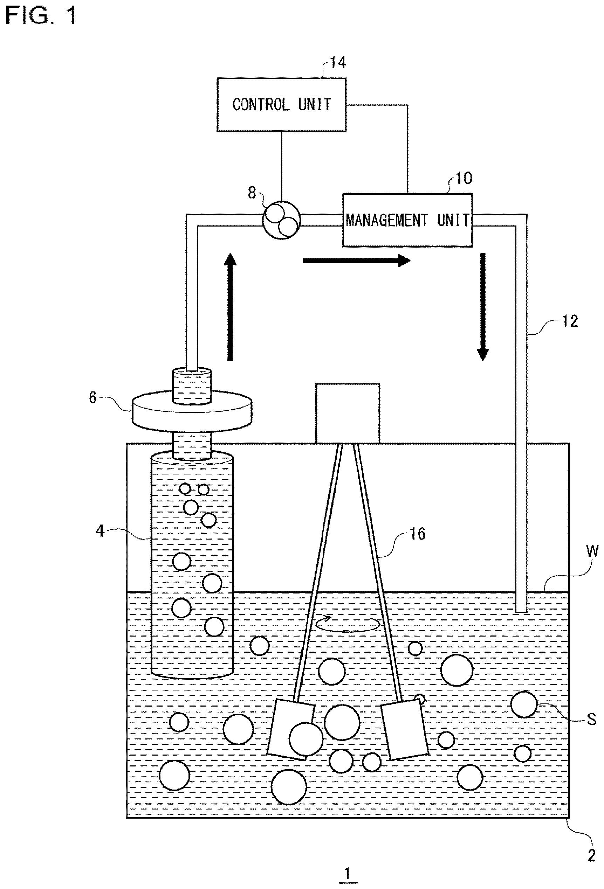

FIG. 1 is a schematic diagram of a culture device 1 according to the first embodiment. The culture device 1 of the present embodiment includes a culture vessel 2, a suction tube 4, an auxiliary filter 6, a suction unit 8, a management unit 10, a discharge pipe 12, and a control unit 14.

The culture vessel 2 is a bioreactor that contains aggregates S of cells and a culture medium W. The culture vessel 2 is not particularly limited as long as it enables suspension culture of the aggregates S. The culture vessel 2 as an example is a so-called spinner flask that includes a stirring blade 16 for stirring the culture medium W. The culture vessel 2 may also be a culture tank, a cell culture bottle, a cell culture bag, or the like. The capacity of the culture vessel 2 may be, for example, 100 mL to 100 L. The culture vessel 2 of the present embodiment may be a closed system in which atmosphere does not substantially enter from the outside or may be an open system in which atmosphere can enter from the outside. When the culture vessel 2 is a closed system, the concentration of oxygen and carbon dioxide in the culture vessel 2 needs to be controlled. Such concentration control is performed, for example, by the management unit 10 or a gas supply/discharge unit, which is not illustrated. The oxygen concentration is controlled, for example, to 1 to 6 ppm. The carbon dioxide concentration is controlled so that the pH of the culture medium W will be 6.8 to 7.6, for example. When the culture vessel 2 is an open system, performing the concentration control described above is not essential but is preferred.

The cells constituting the aggregates S are not particularly limited. For example, the cells may be: pluripotent stem cells, such as human iPS cells, human ES cells, and human Muse cells; differentiation induction cells derived from pluripotent stem cells, such as human iPS cell-derived nephron progenitor cells; somatic stem cells, such as mesenchymal stem cells (MSCs); progenitor cells derived from somatic stem cells, such as nephron progenitor cells; tissue cells, such as human proximal tubule epithelial cells, human distal tubule epithelial cells, and human collecting duct epithelial cells; antibody-producing cell lines, such as human fetal renal cells (HEK293 cells); or antibody-producing cell lines derived from animals other than humans, such as Chinese hamster ovary cells (CHO cells) and insect cells (SF9 cells).

The culture medium W is not particularly limited and may be appropriately selected from publicly-known culture medium based on the type of cells to be cultured, for example. When proximal tubule cells are cultured, for example, REGM (Lonza Ltd.), EpiCM (ScienCell Research Laboratories, Inc.), Keratinocyte SFM (Life Technologies, Inc.), or the like can be used.

The suction tube 4 suctions the culture medium W in the culture vessel 2. One end of the suction tube 4 is inserted into the culture vessel 2 and comes into contact with the culture medium W in the culture vessel 2. The other end of the suction tube 4 is connected to an inlet port of the management unit 10. The tube diameter of the suction tube 4 is not particularly limited, as long as it is large enough for suctioning of the culture medium W by the suction unit 8.

Midway along the suction tube 4, the auxiliary filter 6 is provided. The auxiliary filter 6 is disposed at a predetermined distance from one end of the suction tube 4. The length from the auxiliary filter 6 to the tip of the suction tube 4 may suitably be a length with which at least the suction flow velocity of the culture medium W in a region preceding the auxiliary filter 6 is not affected by the flow of the culture medium W caused by the stirring in the culture vessel 2 or with which such influence of the flow of the culture medium W becomes as small as possible to make the invention feasible. At least in the region of such length from the tip of the suction tube 4, the suction flow velocity of the culture medium W is adjusted to a flow velocity corresponding to an antagonistic suction flow velocity, which will be described later. This region will be hereinafter referred to as a flow velocity adjustment region. The flow velocity adjustment region can be set as appropriate based on the empirical knowledge of the designer or experiments and the like conducted by the designer. Also, when the auxiliary filter 6 is omitted, the flow velocity adjustment region is defined as a region from the tip of the suction tube 4 in which the suction flow velocity of the culture medium W is not affected by the flow of the culture medium W caused by the stirring in the culture vessel 2 or in which such influence of the flow of the culture medium W becomes as small as possible. For example, in the case of the culture vessel 2 of rotational stirring type, the length from the auxiliary filter 6 to the tip may suitably be 1 cm or longer. The auxiliary filter 6 as an example is disposed in a region extending above the liquid level of the culture medium W or a region extending outside the culture vessel 2, in the suction tube 4. The auxiliary filter 6 may be omitted. Also, the pore size of the auxiliary filter 6 may suitably be at least smaller than the particle size of an aggregate S to be cultured, i.e., an aggregate S to be finally recovered, and may be 1 um to 1000 $\mu$m, for example.

Between the auxiliary filter 6 in the suction tube 4 and the management unit 10, the suction unit 8 is provided. The suction unit 8 generates suction force in the suction tube 4. As the suction unit 8, a publicly-known suction device may be used, e.g., a pump such as a peristaltic pump or a diaphragm pump, or an aspirator. By driving the suction unit 8, the culture medium W is suctioned through one end of the suction tube 4. The suction unit 8 can suction the culture medium W at a flow rate of 0.1 mL/minute to 1 L/minute, for example. The arrangement of the suction unit 8 is not particularly limited, as long as suction force can be generated in the suction tube 4.

The culture medium W suctioned from the culture vessel 2 flows within the suction tube 4 toward the other end and flows into the management unit 10 via the auxiliary filter 6 and the suction unit 8. The management unit 10 performs at least one of analysis or adjustment of the components of the suctioned culture medium W. For example, the management unit 10 may include a publicly-known analyzer, such as a pH meter, a dissolved oxygen meter (DO meter), and a spectrometer, to analyze the components of the culture medium W. Also, the management unit 10 may include, for example, a module for replenishing the culture medium W with a medium component, such as glucose or protein, and oxygen. Further, the management unit 10 may include, for example, a column filled with an adsorbent for waste products, such as lactic acid and ammonia, so as to remove waste products from the culture medium W.

To an outlet port of the management unit 10, one end of the discharge pipe 12 is connected. The other end of the discharge pipe 12 is inserted into the culture vessel 2. The culture medium W that has been subjected to analysis processing or regeneration processing in the management unit 10 is returned to the culture vessel 2 through the discharge pipe 12.

The control unit 14 controls driving of the suction unit 8. The control unit 14 is implemented by an element such as a CPU or memory of a computer or by a circuit as a hardware configuration, and by a computer program or the like as a software configuration. FIG. 1 illustrates the control unit 14 as a functional block implemented by coordination of those components as appropriate. It will be understood by those skilled in the art that this functional block may be implemented in a variety of forms by combinations of hardware and software.

For example, a user of the culture device 1 can select the operation mode of the culture device 1 via an operation panel, not illustrated, or the like. As an example, the operation mode includes a culture medium regeneration mode. The control unit 14 receives a signal indicating the selected operation mode from the operation panel and controls the suction unit 8 according to the operation mode. The control unit 14 controls the suction unit 8 based on the antagonistic suction flow velocity of an aggregate S. The antagonistic suction flow velocity is a velocity at which the sedimentation of an aggregate S due to its own weight or the like is antagonistic to the floating of the aggregate S due to the suction of the culture medium W. The control unit 14 selects the magnitude of the suction flow velocity of the culture medium W with respect to the antagonistic suction flow velocity of an aggregate S, depending on the purpose of the operation mode.

In general, as the particle size of an aggregate S increases, the sedimentation velocity of the aggregate S also increases. Also, as the sedimentation velocity increases, the suction flow velocity that balances with the sedimentation velocity also increases. Therefore, the particle size and the antagonistic suction flow velocity of an aggregate S have a positive correlation. Further, the particle size of an aggregate S also has a positive correlation with the number of culture days (culture time). The relationship between the particle size of an aggregate S and the number of culture days can be set as appropriate depending on the type of cells, culture conditions, and the like, based on the empirical knowledge of the designer or experiments and the like conducted by the designer.

Thus, by counting the number of culture days, the control unit 14 can determine the particle size of an aggregate S contained in the culture vessel 2 and thus the antagonistic suction flow velocity, which serves as a basis for suction force adjustment. For example, the control unit 14 may include a built-in timer so as to count the number of culture days, with a signal indicating the start timing of culture input from an operation panel or the like. Also, the control unit 14 may retain in advance a conversion table that relates the number of culture days to the antagonistic suction flow velocity, so as to determine the antagonistic suction flow velocity from the number of culture days using the conversion table.

The particle size of an aggregate S in the culture medium W may be measured using a publicly-known optical method or the like. The particle size of an aggregate S may also be determined based on a parameter other than the number of culture days that has a correlation with the particle size. Such a parameter may be, for example, the glucose consumption rate, the lactic acid production rate, or the oxygen consumption rate. The control unit 14 can determine the antagonistic suction flow velocity using a conversion table that relates an actual measured value of the particle size or a parameter to the antagonistic suction flow velocity. As an example, the particle size of an aggregate S may be considered as an average of the maximum and minimum values of the distance between two points located on the contour of the aggregate S, in a microscope image of the aggregate S, for example.

When the culture medium regeneration mode is selected, the control unit 14 controls the suction unit 8 so as to suction the culture medium W at a flow velocity equal to or lower than the antagonistic suction flow velocities of aggregates S contained in the culture vessel 2. Accordingly, while the culture medium W is transmitted to the management unit 10, the aggregates S contained in the culture vessel 2 are suppressed from being suctioned into the suction tube 4. Meanwhile, the control unit 14 may constantly perform the analysis processing or regeneration processing of the culture medium W regardless of the mode selection. In either case of performing the culture medium regeneration mode or such constant performing, the control unit 14 may perform feedback control of receiving a signal indicating an analysis result of the culture medium W from the management unit 10 and switching the suspension and resumption of suctioning the culture medium W based on the analysis result.

As described above, the antagonistic suction flow velocity of an aggregate S is determined based on the number of culture days. Meanwhile, a distribution may occur in the particle sizes of the aggregates S. More specifically, within the culture vessel 2 after a certain number of culture days, not only the main group of aggregates S having particle sizes corresponding to the number of culture days, but also aggregates S having smaller particle sizes, aggregates S having larger particle sizes, single cells, debris, and the like may be mixed. If the control unit 14 suctions the culture medium W at a flow velocity equal to the antagonistic suction flow velocity for a particle size of the main group or at a flow velocity with a small difference from the antagonistic suction flow velocity, aggregates S having smaller particle sizes or the like may be suctioned into the suction tube 4. Such aggregates S or the like suctioned into the suction tube 4 are restrained from advancing to the management unit 10 side by the auxiliary filter 6. The pore size of the auxiliary filter 6 may suitably be smaller than a target particle size of the aggregates S in cell culture and may be 1 $\mu$m to 1000 $\mu$m, for example. The target particle size of the aggregates S may be, for example, 100 $\mu$m to 1500 $\mu$m.

In the following, the antagonistic suction flow velocity used as a basis for suction force adjustment will be described in detail. The inventors have determined the antagonistic suction flow velocity according to the following procedure.

Sedimentation test of aggregates S

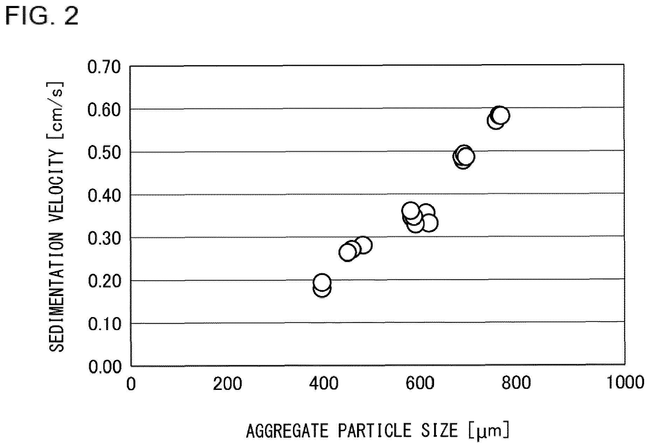

First, a sedimentation test of aggregates S was performed to measure the sedimentation velocities of the aggregates S. In specific, in a 96-well plate {PrimeSurface (registered trademark) Plate 96U, Sumitomo Bakelite Co., Ltd.}, 50 $\mu$l (5000 cells) of mouse embryo-derived nephron progenitor cells (mNPCs) were seeded and cultured using a culture medium for mNPCs. Accordingly, aggregates S of various particle sizes were obtained. The particle sizes of the aggregates S thus obtained were measured using a microscope (BZ-X710, KEYENCE CORPORATION). Thereafter, the aggregates S were gently settled on the culture medium for mNPCs, and time t required for an aggregate S to pass a predetermined drop L was measured. Then, the sedimentation velocity $w_s$ ($w_s$ = L/t) of the aggregate S was calculated. FIG. 2 is a diagram that shows relationships between the particle sizes and the sedimentation velocities of aggregates S. As shown in FIG. 2, it was confirmed that the sedimentation velocity increased as the particle size increased.

Suction test of aggregates S

Next, a suction test of aggregates S was performed to measure the antagonistic suction flow velocities of the aggregates S. In specific, the aggregates S of various particle sizes obtained in the sedimentation test described above were arranged within the suction tube 4, and the culture medium W was suctioned at various suction flow velocities. For the suction tube 4, suction tubes with tube diameters of φ6, φ8, and φ10 (6 mm, 8 mm, and 10 mm) were used. The temperature of the culture medium was set to 25 degrees C. For suction, a peristaltic pump (WPX1, Welco Co., Ltd.) was used. Then, the states of the aggregates S while the culture medium W was being suctioned were visually observed and evaluated. In the evaluation of the states, the case where the aggregate S had sunk was evaluated as "A", the case where the aggregate S had remained, i.e., the case where sinking and floating had been antagonistic, was evaluated as "B", and the case where the aggregate S had floated was evaluated as "C". For each particle size of the aggregates S, the suction flow velocity at which the evaluation B was obtained corresponds to the antagonistic suction flow velocity.

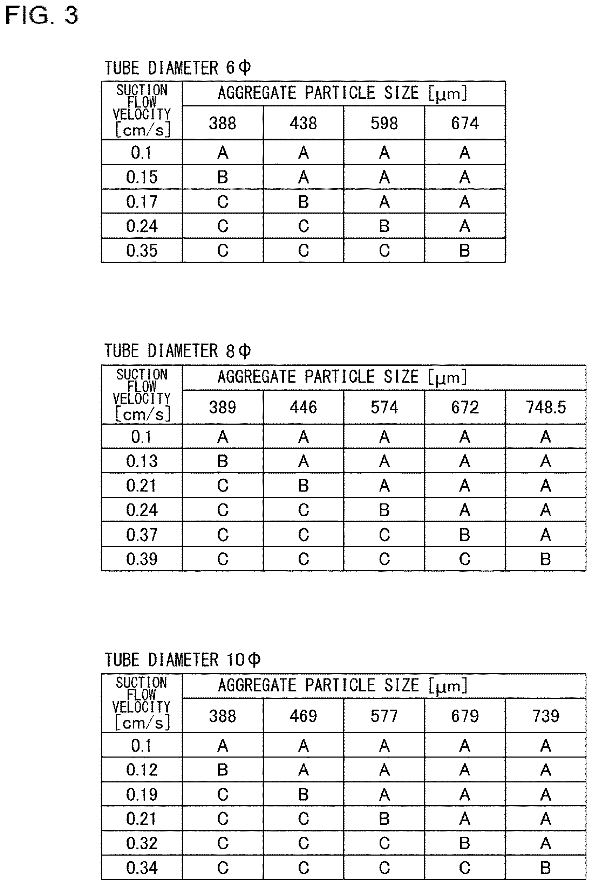

FIG. 3 is a diagram that shows the states of aggregates S when the culture medium W was suctioned at various suction flow velocities. As shown in FIG. 3, it was confirmed that, for any tube diameter of the suction tube 4, aggregates S having similar particle sizes showed similar antagonistic suction flow velocities. Therefore, it was confirmed that, for any tube diameter of the suction tube 4, an aggregate S can be made to sink, placed in the antagonistic state, or made to float by adjusting the suction flow velocity of the culture medium W. In other words, it was shown that, by adjusting the suction flow velocity of the culture medium W, the culture medium W can be transmitted outside the culture vessel 2 without suctioning the aggregates S to be cultured. The suction flow rate of the culture medium W is a value obtained by multiplying the suction flow velocity by the tube diameter (inner diameter) of the suction tube 4. Therefore, by increasing the tube diameter of the suction tube 4, the suction flow rate can be increased while restraining the suction of the aggregates S to be cultured.

Comparison between sedimentation velocity and antagonistic suction flow velocity

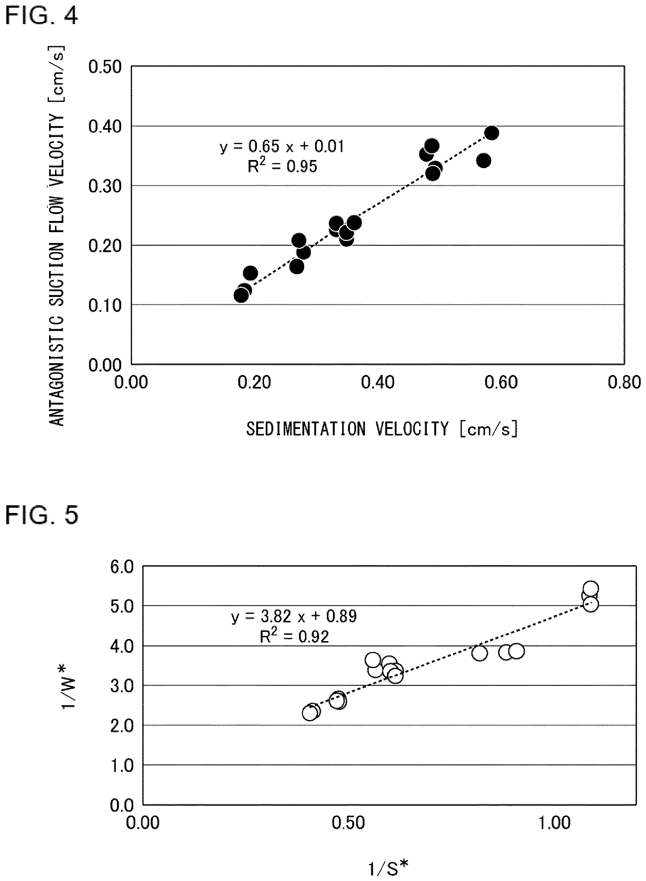

From the results of the sedimentation test and the suction test described above, the relationships between the sedimentation velocities and the antagonistic suction flow velocities of aggregates S were derived. FIG. 4 is a diagram that shows relationships between the sedimentation velocities and the antagonistic suction flow velocities of aggregates S. As shown in FIG. 4, on coordinates in which the sedimentation velocity was set on the first axis (horizontal axis) and the antagonistic suction flow velocity was set on the second axis (vertical axis), the results for each particle size of the aggregates S were plotted and linearly approximated, so as to obtain a linear approximate equation that shows the relationships between the sedimentation velocity and the antagonistic suction flow velocity.

Theoretically, if the suction flow velocity of the culture medium W and the sedimentation velocity of an aggregate S are the same value, the aggregate S should be in an antagonistic state, without sinking or floating. However, in practice, it was confirmed that, although the sedimentation velocity and the antagonistic suction flow velocity of an aggregate S have a positive correlation, the antagonistic suction flow velocity is lower than the sedimentation velocity. This is presumably due to the influence of pulsation of a pump constituting the suction unit 8, for example.

Creation of sedimentation velocity prediction curve

If it is possible to predict the sedimentation velocity for each particle size of the aggregates S and also predict the antagonistic suction flow velocity based thereon, the suction flow velocity of the culture medium W suitable for the purpose can be accurately determined for any particle size of the aggregates S. Therefore, a sedimentation velocity prediction curve for the aggregates S was created, assuming that each aggregate S of mNPCs is a spherical particle. For the creation, a method proposed in the following paper was used: Jimenez, J. A. and Madsen, O. S., A simple formula to estimate settling velocity of natural sediments, Journal of Waterway, Port, Coastal, and Ocean Engineering, Vol.129, No. 2, pp.70-78, 2003.

First, the following Equation (1)
Math. 1

$$W_* = \frac{w_s}{\sqrt{(s-1)gd}} \qquad (1)$$

where $W_*$ is a dimensionless sedimentation velocity, $w_s$ is the sedimentation velocity of an aggregate S [m/s], s is specific gravity given by $\rho_s/\rho_w$, $\rho_s$ is density of the aggregate S [kg/m$^3$], $\rho_w$ is density of the culture medium W [kg/m$^3$], g is the gravitational acceleration [m/s$^2$], and d is the particle size of the aggregate S [m],

was used to convert the sedimentation velocity of each particle size obtained in the sedimentation test described above to a dimensionless sedimentation velocity $W_*$. Equation (1) is a variant of the first equation in the paper (equilibrium equation) and corresponds to the second equation in the paper.

Also, the following Equation (2)

Math. 2

$$S_* = \frac{d}{4v}\sqrt{(s-1)gd} \qquad (2)$$

where $S_*$ is a dimensionless particle parameter, $v$ is kinematic viscosity of the culture medium W given by $\mu/\rho_w$ [m²/s], $\mu$ is viscosity of the culture medium W [Pa·s], $\rho_w$ is density of the culture medium W [kg/m³], and d, s, and g are the same as in Equation (1),

was used to calculate the dimensionless particle parameter $S_*$ for each particle size of the aggregates S. Equation (2) corresponds to the fourth equation in the paper.

Further, the following Equation (3)

Math. 3

$$\frac{1}{W_*} = A + \frac{B}{S_*} \qquad (3)$$

where $W_*$ is the same as in Equation (1), $S_*$ is the same as in Equation (2), and A and B are constants determined from a straight line obtained by plotting, on coordinates in which $1/W_*$ is set on the first axis and $1/S_*$ is set on the second axis, the value for each particle size of the aggregates S,

was used, and, by substituting $W_*$ and $S_*$ for each particle size of the aggregates S into Equation (3), the constants A and B were obtained. Equation (3) corresponds to the sixth equation in the paper. In specific, as shown in FIG. 5, on coordinates in which $1/S_*$ is set on the first axis and $1/W_*$ is set on the second axis, the value for each particle size of the aggregates S was plotted and linearly approximated, so as to obtain a linear approximate equation. Accordingly, the constant A can be obtained from the intercept of the linear approximate equation, and the constant B can be obtained from the slope of the linear approximate equation. FIG. 5 is a diagram that shows relationships between $1/S_*$ and $1/W_*$.

In the series of calculations described above, the gravitational acceleration g was set to 9.80655 m/s², the density $\rho_s$ of the aggregate S was set to 1030 kg/m³, the density $\rho_w$ of the culture medium W at 25 degrees C was set to 1005.294 kg/m³, and the viscosity $\mu$ of the culture medium W at 25 degrees C was set to 1.027 mPa·s. The viscosity $\mu$ of the culture medium W can be measured using a tuning fork vibro viscometer (SV-A, A&D Company, Limited). As a result, in the example shown in FIG. 5, A was 0.89 and B was 3.82 in Equation (3).

For the calculation of the density $\rho_w$ of the culture medium W, specific gravity $s_w$ of the culture medium W at t degrees C was calculated using the following method. The weight $W_1$ [g] of a dry specific gravity bottle (1-4566-01, AS ONE CORPORATION), the weight $W_2$ [g] of a specific gravity bottle filled with pure water at t degrees C, and the weight $W_3$ [g] of a specific gravity bottle filled with the culture medium W at t degrees C were measured with an electronic balance (HR251-AZ, A&D Company, Limited). The density of air at t degrees C was defined as $\rho_{air}$ [g/cm³] and the density of water at 4 degrees C was defined as $\rho_0$ [g/cm³], and the following Equation (F1), provided in the Methods of Measuring Specific Gravity of Liquid (JIS) (Draft), Measurement, Vol. 9, No. 10,

Math. 4

$$s_w = \left\{ \frac{W_3 - W_1}{W_2 - W_1}(\rho_0 - \rho_{air}) + \rho_{air} \right\} \cdot \frac{1}{\rho_0} \qquad (F1)$$

was used to calculate the specific gravity $s_w$ of the culture medium W.

Also, for the calculation of the density $\rho_s$ of an aggregate S, specific gravity $s_s$ of the aggregate S was calculated using the following method. The weight $W_4$ [g] of a dry microtube (130-806C, WATSON Co., Ltd.) was measured with an electronic balance (HR251-AZ, A&D Company, Limited). Then, multiple aggregates S were collected in the microtube. After the microtube was subjected to centrifugation, the supernatant medium was removed, and the weight $W_5$ [g] was measured. Then, the region occupied by the cells was replaced with pure water at t degrees C, and the weight $W_6$ [g] was measured. The density of water at t degrees C was defined as $\rho_1$ [g/cm³], and the following Equation (F2)

Math. 5

$$s_s = \frac{W_5 - W_4}{(W_6 - W_4)/\rho_1} \cdot \frac{1}{\rho_0} \qquad (F2)$$

was used to calculate the specific gravity $s_s$ of the aggregates S.

Equation (F2) is based on a relation of (specific gravity of an object) = (mass of the object) / (mass of water at 4 degrees C of which the volume is the same as the volume of the object), which is a basic method for obtaining specific gravity. It is difficult to measure the weight of water while the temperature is controlled at 4 degrees C. Therefore, the volume of water at room temperature is calculated by dividing the weight of water measured in a stable state at room temperature by the density of water at the same temperature. Thereafter, by dividing the weight of the aggregates S by the volume of water at room temperature and then by the density of water at 4 degrees C, the specific gravity $s_s$ of the aggregates S can be calculated.

Subsequently, the following Equation (4), obtained by substituting Equation (1) and Equation (2) into Equation (3),

Math. 6

$$w_s = \frac{(s-1)gd^2}{4vB + Ad\sqrt{(s-1)gd}} \qquad (4)$$

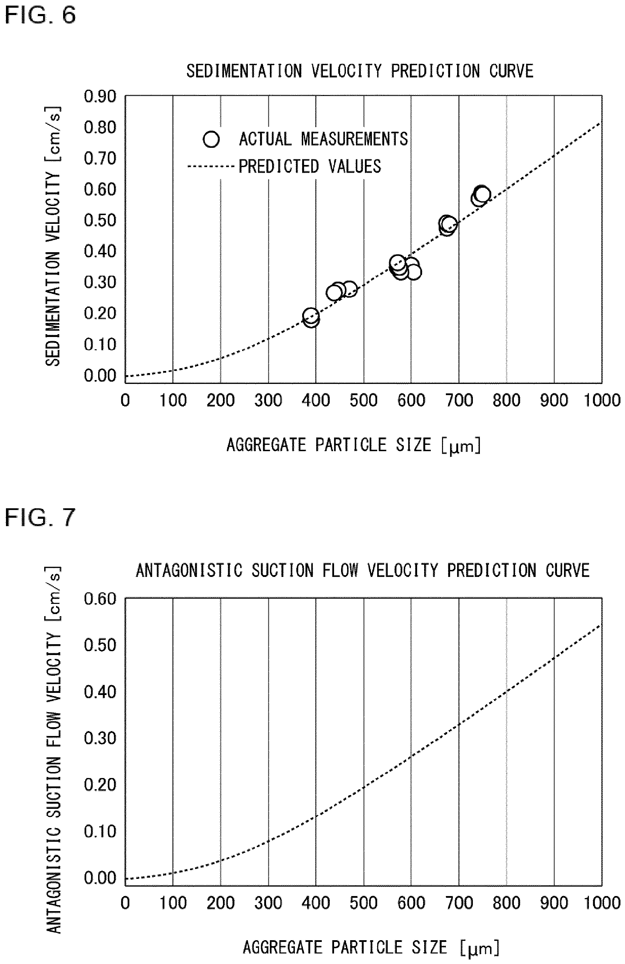

where $w_s$, $s$, $g$, and $d$ are the same as in Equation (1), $v$ is the same as in Equation (2), and A and B are the same as in Equation (3),

was used and, by substituting the values of A and B into Equation (4), a sedimentation velocity prediction curve for the aggregates S could be obtained. FIG. 6 is a diagram that shows a sedimentation velocity prediction curve. As shown in FIG. 6, it was confirmed that, when the actual measured values of the sedimentation velocity obtained in the sedimentation test of the aggregates S described above and the sedimentation velocity prediction curve were compared, both were approximate with high accuracy.

Creation of antagonistic suction flow velocity prediction curve

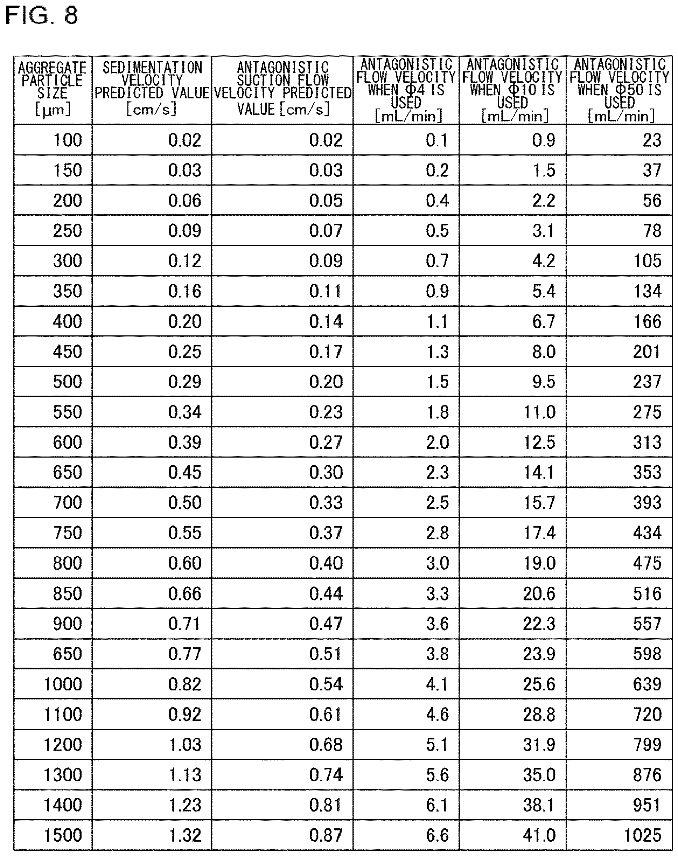

Then, using the aforementioned linear approximate equation that shows the relationships between the sedimentation velocity and the antagonistic suction flow velocity, the sedimentation velocity prediction curve obtained from Equation (4) is converted into an antagonistic suction flow velocity prediction curve. Based on the antagonistic suction flow velocity determined based on the sedimentation velocity prediction curve, the control unit 14 adjusts the suction flow velocity force of the culture medium W generated by the suction unit 8. FIG. 7 is a diagram that shows an antagonistic suction flow velocity prediction curve. Also, FIG. 8 is a diagram that shows a predicted value of the sedimentation velocity, a predicted value of the antagonistic suction flow velocity, and an antagonistic flow rate at each tube diameter, for each particle size of the aggregates S. It was confirmed that, when the actual measured values of the antagonistic suction flow velocity obtained in the suction test of the aggregates S described above and the antagonistic suction flow velocity prediction curve were compared, both were approximate with high accuracy.

As described above, the culture device 1 according to the present embodiment includes: the culture vessel 2 that contains an aggregate S of cells and the culture medium W; the suction tube 4 that suctions the culture medium W in the culture vessel 2; the suction unit 8 that generates suction force in the suction tube 4; and the control unit 14 that controls the suction unit 8 based on the antagonistic suction flow velocity of the aggregate S, which is a velocity at which sedimentation of the aggregate S is antagonistic to floating of the aggregate S due to the suction of the culture medium W. Accordingly, operations such as suctioning only the culture medium W (i.e., separation of the culture medium W and the aggregates S) and classification of the aggregates S can be performed without using a filter. Therefore, since adhering of the aggregates S to the filter can be prevented, the efficiency of cell culture can be improved.

Also, the control unit 14 of the present embodiment controls the suction unit 8 so as to suction the culture medium W at a flow velocity equal to or lower than the antagonistic suction flow velocity. This enables separation of the aggregates S and the culture medium W without using a filter. Therefore, the efficiency of cell culture can be improved.

Also, in the present embodiment, the antagonistic suction flow velocity is determined based on a sedimentation velocity prediction curve for the aggregates S obtained from Equations (1) to (4) described above. This enables the suction of the culture medium W while restraining the suction of the aggregates S, for any particle size of the aggregates S. Further, depending on the purpose of suctioning the culture medium W, a more suitable suction flow velocity of the culture medium W can be set.

The culture device 1 of the present embodiment also includes the management unit 10 that is connected to the suction tube 4 and that performs at least one of analysis or adjustment of the components of the suctioned culture medium W. Accordingly, the states of the cells and the culture medium W can be grasped more easily, and a more favorable culture environment can be created. Therefore, the efficiency of cell culture can be further improved.

Second Embodiment

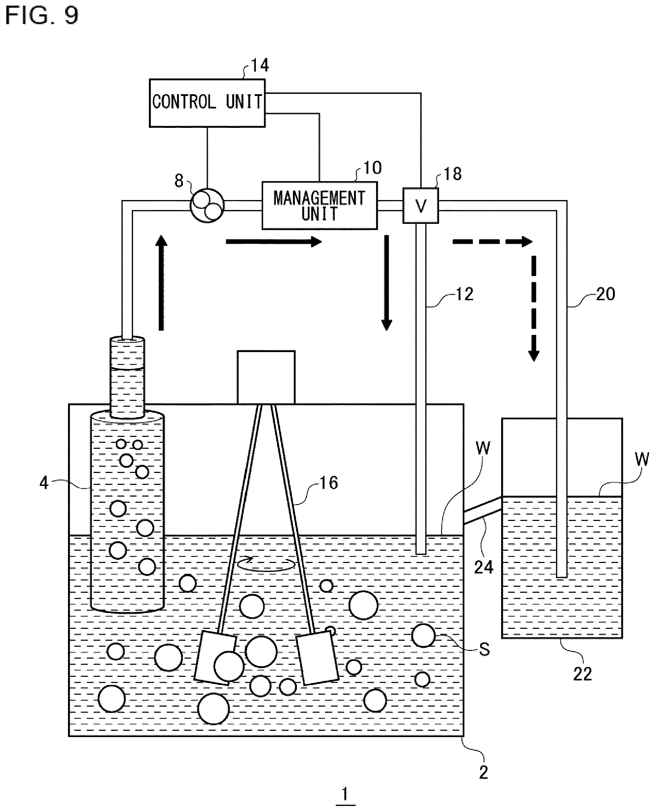

The second embodiment includes configurations in common with the first embodiment, except for part of the configuration of the culture device 1 and the content of control performed by the control unit 14. In the following, the present embodiment will be described mainly for configurations different from those in the first embodiment, and description of configurations in common will be briefly given or may be omitted. FIG. 9 is a schematic diagram of a culture device 1 according to the second embodiment. The culture device 1 of the present embodiment further includes a switching valve 18, a recovery pipe 20, and a recovery container 22, in addition to the culture vessel 2, suction tube 4, suction unit 8, management unit 10, discharge pipe 12, and control unit 14. The auxiliary filter 6 is omitted.

To the outlet port of the management unit 10, one end of the discharge pipe 12 and one end of the recovery pipe 20 are connected via the switching valve 18. The other end of the discharge pipe 12 is inserted into the culture vessel 2. Also, the other end of the recovery pipe 20 is inserted into the recovery container 22. The switching valve 18 may be constituted by, for example, a publicly-known solenoid valve and can switch the destination of the culture medium W flowing out from the outlet port of the management unit 10, between the discharge pipe 12 and the recovery pipe 20. When the switching valve 18 is made to set the destination of the culture medium W to the discharge pipe 12, the culture medium W that has passed through the management unit 10 is returned to the culture vessel 2 through the discharge pipe 12. When the switching valve 18 is made to set the destination of the culture medium W to the recovery pipe 20, the culture medium W that has passed through the management unit 10 is transmitted to the recovery container 22 through the recovery pipe 20. Each of the culture vessel 2 and the recovery container 22 of the present embodiment may be a closed system in which atmosphere does not substantially enter from the outside or may be an open system in which atmosphere can enter from the outside. When the culture vessel 2 and the recovery container 22 are closed systems, the concentration control described previously is performed for oxygen and carbon dioxide in the culture vessel 2 and the recovery container 22. When the culture vessel 2 and the recovery container 22 are open systems, performing the concentration control is not essential but is preferred.

The culture device 1 also includes a pipe 24 that connects the culture vessel 2 and the recovery container 22. The supernatant that does not contain the aggregates S in the culture medium W held in the recovery container 22 is returned to the culture vessel 2 through the pipe 24. This can secure an amount of the culture medium W in the culture vessel 2. Such transfer from the recovery container 22 to the culture vessel 2 through the pipe 24 may be implemented by using the difference in height of the liquid level of the culture medium W in the recovery container 22 and the culture vessel 2, as shown in FIG. 9, or may be implemented by providing a suction unit, such as a pump, in the pipe 24. When a suction unit is provided in the pipe 24, the arrangement of the pipe 24 is not limited to that shown in FIG. 9.

The control unit 14 controls the driving of the suction unit 8 and the switching of the switching valve 18. As an example, the culture device 1 of the present embodiment can select a medium regeneration mode, an aggregate disposal mode, and an aggregate recovery mode. The control unit 14 controls the suction unit 8 and the switching valve 18 depending on each operation mode.

When the culture medium regeneration mode is selected, the control unit 14 controls the suction unit 8 so as to suction the culture medium W at a flow velocity equal to or lower than the antagonistic suction flow velocities of aggregates S contained in the culture vessel 2, as described in the first embodiment. The control unit 14 also controls the switching valve 18 so that the culture medium W flows into the discharge pipe 12. Accordingly, the culture medium W that has been subjected to regeneration processing in the management unit 10 is returned to the culture vessel 2.

When the aggregate disposal mode is selected, the control unit 14 controls the suction unit 8 so as to suction the culture medium W at a first flow velocity that is higher than the antagonistic suction flow velocity for an aggregate S having a predetermined first particle size and that is equal to or lower than the antagonistic suction flow velocity for an aggregate S having a second particle size larger than the first particle size. The first particle size is the particle size of the largest aggregate S among aggregates S to be disposed of. The second particle size is the particle size of an aggregate S to be cultured. The control unit 14 also controls the switching valve 18 so that the culture medium W flows into the recovery pipe 20. Accordingly, the aggregates S to be cultured and the aggregates S to be disposed of can be sorted (classified), and the aggregates S to be disposed of can be transferred from the culture vessel 2 to the recovery container 22. The aggregates S to be disposed of held in the recovery container 22 are disposed of outside the device.

In the culture medium regeneration mode, even the aggregates S to be disposed of may desirably be suctioned as few as possible. On the other hand, in the aggregate disposal mode, the aggregates S to be disposed of should be actively suctioned. Therefore, the control unit 14 as an example retains in advance, with respect to the flow velocity, a predetermined first margin M1, and a second margin M2 smaller than the first margin M1. Then, from the antagonistic suction flow velocity as a basis determined based on the number of culture days and the like, a margin selected depending on the mode to be executed is subtracted, so as to determine the flow velocity to be generated by the suction unit 8.

In specific, when the culture medium regeneration mode is selected, the suction unit 8 is controlled to suction the culture medium W at a flow velocity obtained by subtracting the larger first margin M1 from the antagonistic suction flow velocity. This can reduce the suctioned amount of aggregates S to be disposed of. Meanwhile, in the aggregate disposal mode, the suction unit 8 is controlled to suction the culture medium W at a flow velocity obtained by subtracting the smaller second margin M2 from the antagonistic suction flow velocity. In other words, the first flow velocity is determined by subtracting the second margin M2 from the antagonistic suction flow velocity. Accordingly, the aggregates S to be disposed of can be suctioned more certainly. The first margin M1 and the second margin M2 can be set as appropriate based on the empirical knowledge of the designer or experiments and the like conducted by the designer.

Further, when the aggregate recovery mode is selected, the control unit 14 controls the suction unit 8 so as to suction the culture medium W at the first flow velocity and then suction the culture medium W at a second flow velocity that is higher than the antagonistic suction flow velocity for an aggregate S having the second particle size. In other words, the aggregate recovery mode includes, as part thereof, the processing performed in the aggregate disposal mode. The control unit 14 also controls the switching valve 18 so that the culture medium W flows into the recovery pipe 20. Accordingly, an aggregate S that has grown enough to be recovered, i.e., an aggregate S having the second particle size, can be transferred from the culture vessel 2 to the recovery container 22. Such an aggregate S having the second particle size can be isolated by transferring, to separate recovery containers 22, the culture medium W suctioned at the first flow velocity and the culture medium W suctioned at the second flow velocity. The aggregates S held in the recovery container 22 are used as intended. In order that an aggregate S to be recovered may be suctioned more certainly, the second flow velocity is set to a value obtained by adding a predetermined third margin M3 to the antagonistic suction flow velocity for the aggregate S to be recovered. The third margin M3 can be set as appropriate based on the empirical knowledge of the designer or experiments and the like conducted by the designer.

In the aggregate recovery mode, it is suitable to suction the culture medium W using the suction unit 8 constituted by an aspirator rather than a pump. This can prevent the collapse of the aggregates S to be recovered during transfer to the recovery container 22. Also, in the culture device 1 where disposal or recovery of aggregates is performed, the tube diameter of the suction tube 4 may suitably be larger. This can improve the suction separation capability for the aggregates S. For example, the sedimentation velocity of an aggregate S of which the particle size is several tens of micrometers to 2 mm is about 0.01 cm/second to 2 cm/second. When classification between such an aggregate S and other aggregates S is performed at a flow rate of 0.1 mL/minute or more, the tube diameter may suitably be φ4 or larger. Also, when classification between such an aggregate S and other aggregates S is performed at a flow rate of 1 L/minute or less, the tube diameter may suitably be φ50 or smaller.

The flow path structure for transmitting the suctioned culture medium W to the recovery container 22 is not limited to that shown in FIG. 9. Also, the auxiliary filter 6 may be provided in the suction tube 4 without being omitted. In this case, the culture device 1 as an example may include a suction tube 4 used in the culture medium regeneration mode, a suction tube 4 used in the aggregate disposal mode, and a suction tube 4 used in the aggregate recovery mode, separately. Each of the suction tubes 4 is provided with an auxiliary filter 6 having a pore size appropriate for the operation mode. The suction tube 4 used in the culture medium regeneration mode is provided with an auxiliary filter 6 similar to that in the first embodiment. The suction tube 4 used in the aggregate disposal mode is provided with an auxiliary filter 6 through which the aggregates S to be disposed of can pass. The pore size of this auxiliary filter 6 may be set in the range of 100 μm to 1500 μm, for example. The suction tube 4 used in the aggregate recovery mode is provided with an auxiliary filter 6 through which the aggregates S to be recovered can pass. The pore size of this auxiliary filter 6 may be set in the range of 100 μm to 1500 μm, for example.

As described above, the control unit 14 according to the present embodiment controls the suction unit 8 so as to suction the culture medium W at the first flow velocity that is higher than the antagonistic suction flow velocity for an aggregate S having the predetermined first particle size and that is equal to or lower than the antagonistic suction flow velocity for an aggregate S having the second particle size larger than the first particle size. This enables classification between aggregates S having the first particle size and aggregates S having the second particle size. Accordingly, when aggregates S having the first particle size are set as those to be disposed of and aggregates S having the second particle size are set as those to be cultured, the aggregates S to be disposed of can be disposed of more easily. This enables culture of the aggregates S to be cultured in a more favorable environment, so that the efficiency of cell culture can be improved.

The control unit 14 also controls the suction unit 8 so as to suction the culture medium W at the first flow velocity and then suction the culture medium W at the second flow velocity that is higher than the antagonistic suction flow velocity for an aggregate S having the second particle size. Accordingly, the target aggregates S can be recovered more easily. Therefore, the efficiency of cell culture can be improved.

Third Embodiment

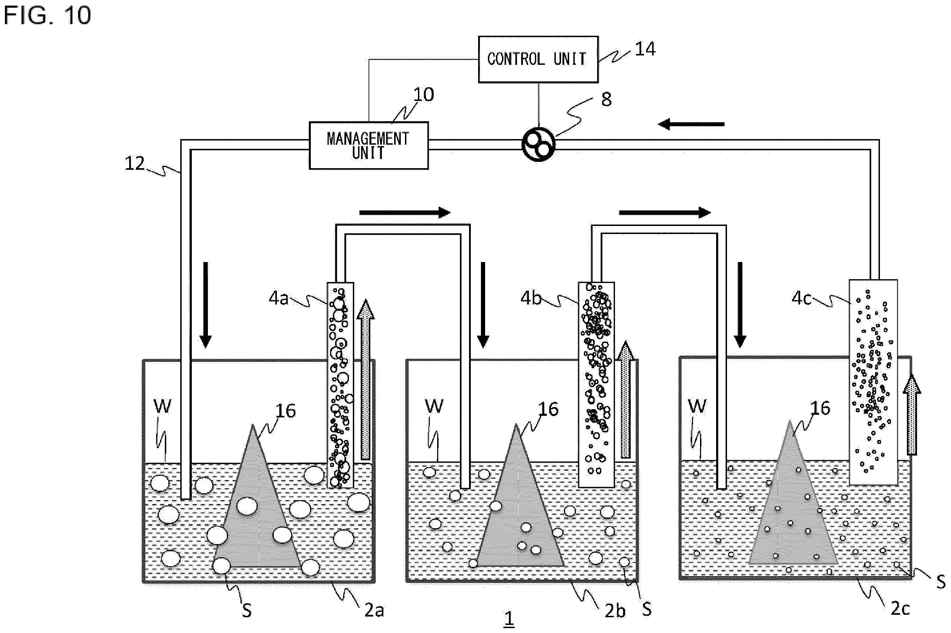

The third embodiment includes configurations in common with the first embodiment, except that the culture device 1 includes multiple culture vessels 2 and multiple suction tubes 4. In the following, the present embodiment will be described mainly for configurations different from those in the first embodiment, and description of configurations in common will be briefly given or may be omitted. FIG. 10 is a schematic diagram of a culture device 1 according to the third embodiment. The culture device 1 of the present embodiment includes a first culture vessel 2a, a second culture vessel 2b, a third culture vessel 2c, a first suction tube 4a, a second suction tube 4b, a third suction tube 4c, the suction unit 8, the management unit 10, the discharge pipe 12, and the control unit 14. The auxiliary filter 6 is omitted.

One end of the first suction tube 4a is inserted into the first culture vessel 2a. The other end of the first suction tube 4a is inserted into the second culture vessel 2b. Also, one end of the second suction tube 4b is inserted into the second culture vessel 2b. The other end of the second suction tube 4b is inserted into the third culture vessel 2c. Also, one end of the third suction tube 4c is inserted into the third culture vessel 2c. The other end of the third suction tube 4c is connected to the inlet port of the management unit 10. Midway along the third suction tube 4c, the suction unit 8 is provided. Each culture vessel is provided as a closed system in which atmosphere does not substantially enter from the outside. Therefore, by driving the suction unit 8, suction force can be generated in the first suction tube 4a to the third suction tube 4c. To the outlet port of the management unit 10, one end of the discharge pipe 12 is connected. The other end of the discharge pipe 12 is inserted into the first culture vessel 2a. Since each culture vessel is a closed system, the concentration control described previously is performed for oxygen and carbon dioxide in each culture vessel.

By driving the suction unit 8, the first suction tube 4a suctions the culture medium W from the first culture vessel 2a. The culture medium W suctioned from the first culture vessel 2a is transferred to the second culture vessel 2b through the first suction tube 4a. Also, with the suction unit 8 driven, the second suction tube 4b suctions the culture medium W from the second culture vessel 2b. The culture medium W suctioned from the second culture vessel 2b is transferred to the third culture vessel 2c through the second suction tube 4b. Further, with the suction unit 8 driven, the third suction tube 4c suctions the culture medium W from the third culture vessel 2c. The culture medium W suctioned from the third culture vessel 2c flows into the management unit 10 through the third suction tube 4c. The culture medium W that has passed through the management unit 10 is returned to the first culture vessel 2a through the discharge pipe 12. The arrangement of the suction unit 8 and the management unit 10 can be changed as appropriate. For example, the suction unit 8 and the management unit 10 may be provided in the first suction tube 4a or the second suction tube 4b.

The first suction tube 4a, the second suction tube 4b, and the third suction tube 4c have different tube diameters from each other at least in the flow velocity adjustment region. Therefore, by driving the suction unit 8, a suction flow velocity (or linear velocity) of different magnitude is generated in each suction tube 4. For example, a suction tube 4 positioned on the upstream side of the flow of the culture medium W has a smaller tube diameter. That is, the first suction tube 4a has the smallest tube diameter, the second suction tube 4b has an intermediate tube diameter, and the third suction tube 4c has the largest tube diameter. Accordingly, the fastest suction flow velocity occurs in the first suction tube 4a, an intermediate suction flow velocity occurs in the second suction tube 4b, and the slowest suction flow velocity occurs in the third suction tube 4c. Therefore, the first suction tube 4a can suction an aggregate S of larger particle size than the second suction tube 4b and the third suction tube 4c. Also, the second suction tube 4b can suction an aggregate S of larger particle size than the third suction tube 4c.

Thus, aggregates S with relatively large particle sizes are collected in the first culture vessel 2a, aggregates S with relatively intermediate particle sizes are collected in the second culture vessel 2b, and aggregates S with relatively small particle sizes are collected in the third culture vessel 2c. The particle size of aggregates S to be collected in each culture vessel 2 can be changed as appropriate by adjusting the tube diameter of each suction tube 4 or the output of the suction device constituting the suction unit 8, for example.

Thus, the culture device 1 of the present embodiment has multiple combinations of the culture vessel 2 and the suction tube 4. The multiple combinations are connected in series with each other. Also, the tube diameters of the suction tubes 4 are different from each other. By driving the suction unit 8, the culture medium W is suctioned by the suction tubes 4 and sequentially transferred from the culture vessel 2 on the upstream side to the culture vessel 2 on the downstream side. Accordingly, in each of the multiple culture vessels 2, aggregates S having particle sizes corresponding to the tube diameter of the corresponding suction tube 4 can be collected. Therefore, the efficiency of cell culture can be further improved. The number of pairs of the culture vessel 2 and the suction tube 4 may be two or may be four or more.

Fourth Embodiment

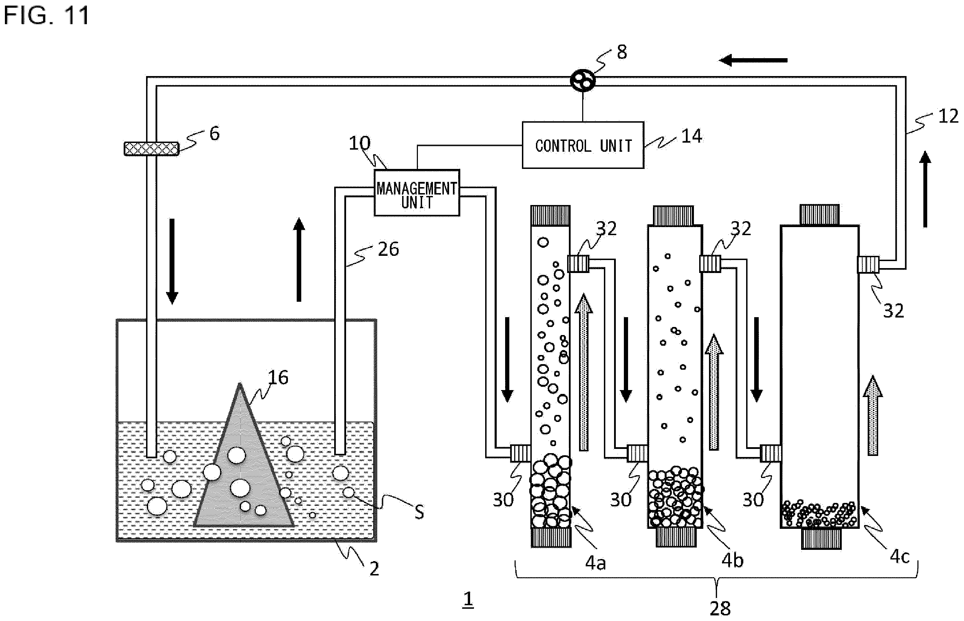

The fourth embodiment includes configurations in common with the first embodiment, except that a recovery unit constituted by a suction tube 4 is provided. In the following, the present embodiment will be described mainly for configurations different from those in the first embodiment, and description of configurations in common will be briefly given or may be omitted. FIG. 11 is a schematic diagram of a culture device 1 according to the fourth embodiment. The culture device 1 of the present embodiment includes the culture vessel 2, the suction unit 8, the management unit 10, the discharge pipe 12, the control unit 14, a connection pipe 26, and a recovery unit 28 (trap chamber).

The connection pipe 26 connects the culture vessel 2 and the recovery unit 28. One end of the connection pipe 26 is inserted into the culture vessel 2. The culture vessel 2 of the present embodiment may be a closed system in which atmosphere does not substantially enter from the outside or may be an open system in which atmosphere can enter from the outside. When the culture vessel 2 is a closed system, the concentration control described previously is performed for oxygen and carbon dioxide in the culture vessel 2. When the culture vessel 2 is an open system, performing the concentration control is not essential but is preferred. The other end of the connection pipe 26 is connected to an inlet port of the recovery unit 28. To an outlet port of the recovery unit 28, one end of the discharge pipe 12 is connected. The other end of the discharge pipe 12 is inserted into the culture vessel 2. The management unit 10 is provided midway along the connection pipe 26. Also, the suction unit 8 is provided midway along the discharge pipe 12. Further, the auxiliary filter 6 is provided downstream of the suction unit 8 in the discharge pipe 12.

By driving the suction unit 8, the culture medium W in the culture vessel 2 is transferred to the recovery unit 28 through the connection pipe 26. In this process, the culture medium W is subjected to regeneration processing or the like in the management unit 10. The culture medium W that has reached the recovery unit 28 is subjected to recovery processing for aggregates S, as described later, in the recovery unit 28. Thereafter, the culture medium W is returned to the culture vessel 2 through the discharge pipe 12. The arrangement of the suction unit 8 and the management unit 10 can be changed as appropriate. For example, the suction unit 8 may be provided in the connection pipe 26, and the management unit 10 may be provided in the discharge pipe 12.

However, the suction unit 8 may suitably be provided downstream of the recovery unit 28, i.e., in the discharge pipe 12. This can avoid the aggregates S from passing through the suction unit 8. In a flow path structure in which aggregates S pass through the suction unit 8, there is a risk that some aggregates S may be destroyed when passing through the suction unit 8. In contrast, by disposing the suction unit 8 downstream of the recovery unit 28, the destruction of aggregates S in the suction unit 8 can be avoided. Or, although measures against the destruction of aggregates S in the suction unit 8 may be necessary in a flow path structure in which aggregates S pass through the suction unit 8, such measures can be omitted by disposing the suction unit 8 downstream of the recovery unit 28.

The recovery unit 28 includes a suction tube 4. The recovery unit 28 of the present embodiment includes a first suction tube 4a, a second suction tube 4b, and a third suction tube 4c. Each suction tube 4 is postured to extend in a vertical direction, and each of the upper end and the lower end thereof is closed with a cap. Also, on a side surface of each suction tube 4, an inlet port 30 is provided closer to the lower end, and an outlet port 32 is provided closer to the upper end. Thus, the outlet port 32 is disposed higher than the inlet port 30. To the inlet port 30 of the first suction tube 4a, the other end of the connection pipe 26 is connected. Therefore, the inlet port 30 of the first suction tube 4a corresponds to the inlet port of the recovery unit 28. The outlet port 32 of the first suction tube 4a is connected to the inlet port 30 of the second suction tube 4b via a pipe. Also, the outlet port 32 of the second suction tube 4b is connected to the inlet port 30 of the third suction tube 4c via a pipe. To the outlet port 32 of the third suction tube 4c, one end of the discharge pipe 12 is connected. Therefore, the outlet port 32 of the third suction tube 4c corresponds to the outlet port of the recovery unit 28.

As in the third embodiment, the first suction tube 4a, the second suction tube 4b, and the third suction tube 4c have different tube diameters from each other at least in the flow velocity adjustment region. Therefore, by driving the suction unit 8, a suction flow velocity of different magnitude is generated in each suction tube 4. For example, a suction tube 4 positioned on the upstream side of the flow of the culture medium W has a smaller tube diameter. That is, the first suction tube 4a has the smallest tube diameter, the second suction tube 4b has an intermediate tube diameter, and the third suction tube 4c has the largest tube diameter. Therefore, the fastest suction flow velocity occurs in the first suction tube 4a, an intermediate suction flow velocity occurs in the second suction tube 4b, and the slowest suction flow velocity occurs in the third suction tube 4c. Accordingly, in the first suction tube 4a, aggregates S with relatively intermediate particle sizes and aggregates S with relatively small particle sizes are suctioned up together with the culture medium W to the outlet port 32 and transmitted to the second suction tube 4b. In the second suction tube 4b, aggregates S with relatively small particle sizes are suctioned up together with the culture medium W to the outlet port 32 and transmitted to the third suction tube 4c. In the third suction tube 4c, even aggregates S with relatively small particle sizes are not suctioned up to the outlet port 32, and only the culture medium W flows into the discharge pipe 12.

Accordingly, aggregates S with relatively large particle sizes are trapped in a bottom part of the first suction tube 4a, aggregates S with relatively intermediate particle sizes are trapped in a bottom part of the second suction tube 4b, and aggregates S with relatively small particle sizes are trapped in a bottom part of the third suction tube 4c. The particle size of aggregates S to be trapped in each suction tube 4 can be changed as appropriate by adjusting the tube diameter of each suction tube 4 or the output of the suction device constituting the suction unit 8, for example.

Thus, the culture device 1 of the present embodiment includes the recovery unit 28 including a suction tube 4. The suction tube 4 includes the inlet port 30 and the outlet port 32 disposed higher than the inlet port 30, and the inlet port 30 is connected to the culture vessel 2. By driving the suction unit 8, the culture medium W is suctioned by the suction tube 4 to flow into the suction tube 4 through the inlet port 30 and flows up within the suction tube 4 to be discharged through the outlet port 32. As a result, aggregates S with particle sizes corresponding to the tube diameter are recovered in the suction tube 4.

Further, the recovery unit 28 of the present embodiment includes multiple suction tubes 4 having different tube diameters. The outlet port 32 of one of adjacent two suction tubes 4 is connected to the inlet port 30 of the other thereof, so that the multiple suction tubes 4 are connected in series with each other. The inlet port 30 of the most upstream suction tube 4 is connected to the culture vessel 2. By driving the suction unit 8, the culture medium W is suctioned by the suction tubes 4 and sequentially transferred from the suction tube 4 on the upstream side to the suction tube 4 on the downstream side. The culture medium W flows up within each suction tube 4 from the inlet port to the outlet port and moves to the suction tube 4 on the downstream side. As a result, aggregates S with particle sizes corresponding to the tube diameter are recovered in each suction tube 4. Accordingly, aggregates S with desired particle sizes can be recovered more easily, so that the efficiency of cell culture can be further improved. The number of suction tubes 4 provided in the recovery unit 28 is not particularly limited and may be one or more.

Embodiments of the present invention have been described in detail. The abovementioned embodiments merely describe specific examples for carrying out the present invention. The embodiments are not intended to limit the technical scope of the present invention, and various design modifications, including changes, addition, and deletion of constituting elements, may be made to the embodiments without departing from the spirit of the invention defined in the claims. Such an additional embodiment with a design modification added has the effect of each of the combined embodiments and modifications. In the aforementioned embodiments, matters to which design modifications may be made are emphasized with the expression of "of the present embodiment", "in the present embodiment", or the like. However, design modifications may also be made to matters without such expression. Optional combinations of the abovementioned constituting elements may also be employed as additional aspects of the present invention.

The embodiments may be defined by the following items.

First item

A culture device (1), including:

a culture vessel (2) that contains one or more aggregates (S) of cells and a culture medium (W);
a suction tube (4) that suctions a culture medium (W) in the culture vessel (2);
a suction unit (8) that generates suction force in the suction tube (4); and
a control unit (14) that controls the suction unit (8) based on an antagonistic suction flow velocity of an aggregate (S), which is a velocity at which sedimentation of the aggregate (S) is antagonistic to floating of the aggregate (S) due to the suction of the culture medium (W).

Second item

The culture device (1) according to First item, wherein the antagonistic suction flow velocity is determined based on a sedimentation velocity prediction curve for the aggregates (S) obtained from the Equation (1), the Equation (2), the Equation (3), and the Equation (4) described above.

Third item

The culture device (1) according to First or Second item, wherein the control unit (14) controls the suction unit (8) so as to suction the culture medium (W) at a flow velocity equal to or lower than the antagonistic suction flow velocity.

Fourth item

The culture device (1) according to First or Second item, wherein the control unit (14) controls the suction unit (8) so as to suction the culture medium (W) at a first flow velocity that is higher than the antagonistic suction flow velocity for an aggregate (S) having a predetermined first particle size and that is equal to or lower than the antagonistic suction flow velocity for an aggregate (S) having a second particle size larger than the first particle size.

Fifth item

The culture device (1) according to Fourth item, wherein the control unit (14) controls the suction unit (8) so as to suction the culture medium (W) at the first flow velocity and then suction the culture medium (W) at a second flow velocity that is higher than the antagonistic suction flow velocity for the aggregate (S) having the second particle size.

Sixth item

The culture device (1) according to any one of First through Fifth items, further including a management unit (10) that is connected to the suction tube (4) and that performs at least one of analysis or adjustment of a component of the suctioned culture medium (W).

Seventh item

The culture device (1) according to any one of First through Sixth items, including a plurality of combinations of the culture vessel (2a, 2b, 2c) and the suction tube (4a, 4b, 4c),

wherein the plurality of combinations are connected with each other,
wherein the tube diameters of the suction tubes (4a, 4b, 4c) are different from each other, and
wherein, when the suction unit (8) is driven, the culture medium (W) is suctioned by the suction tubes (4a, 4b, 4c) and sequentially transferred from the culture vessel (2a) on the upstream side to the culture vessel (2c) on the downstream side.

Eighth item

The culture device (1) according to any one of First through Sixth items, further including a recovery unit (28) for the aggregates (S) including the suction tube (4),

wherein the suction tube (4) includes an inlet port (30) and an outlet port (32) disposed higher than the inlet port (30), and the inlet port (30) is connected to the culture vessel (2), and
wherein, when the suction unit (8) is driven, the culture medium (W) is suctioned by the suction tube (4) to flow into the suction tube (4) through the inlet port (30) and flows up within the suction tube (4) to be discharged through the outlet port (32), so that, in the suction tube (4), an aggregate (S) with a particle size corresponding to the tube diameter is recovered.

Ninth item

The culture device (1) according to Eighth item,

wherein the recovery unit (28) includes a plurality of suction tubes (4a, 4b, 4c) having different tube diameters,
wherein the outlet port (32) of one of adjacent two of the suction tubes (4a, 4b, 4c) is connected to the inlet port (30) of the other thereof, so that the plurality of suction tubes (4a, 4b, 4c) are connected with each other, and the inlet port (30) of the most upstream suction tube (4a) is connected to the culture vessel (2), and
wherein, when the suction unit (8) is driven, the culture medium (W) is suctioned by the suction tubes (4a, 4b, 4c) and sequentially transferred from the suction tube (4a) on the upstream side to the suction tube (4c) on the downstream side, so that, in each of the suction tubes (4a, 4b, 4c), an aggregate (S) with a particle size corresponding to the tube diameter is recovered.

Tenth item

A culture method, including suctioning, from a culture vessel (2) that contains one or more aggregates (S) of cells and a culture medium (W), the culture medium (W) at a flow velocity corresponding to an antagonistic suction flow velocity of an aggregate (S), which is a velocity at which sedimentation of the aggregate (S) is antagonistic to floating of the aggregate (S) due to the suction of the culture medium (W).

Eleventh item

The culture method according to Tenth item, wherein the antagonistic suction flow velocity is determined based on a sedimentation velocity prediction curve for the aggregates (S) obtained from the Equation (1), the Equation (2), the Equation (3), and the Equation (4) described above.

Example

An example of the present invention will now be described by way of example only to suitably describe the present invention and should not be construed as limiting the present invention.

Cell culture test

Comparative Example 1

Using a publicly-known culture vessel with a suction tube, stirred suspension culture of mNPCs was conducted at an initial cell density of $1 \times 10^5$ cells/mL. The tube diameter of the suction tube was $\varphi10$. On the second day of culture when the particle sizes of many aggregates became 200 $\mu$m or larger, a filter with a pore size of 100 $\mu$m was installed at the tip of the suction tube. Then, suction of the culture medium was started at a flow rate of 1 mL/minute, and the culture was continued while the suctioned culture medium was returned to the culture vessel. As shown in FIG. 8, when the tube diameter of the suction tube is $\varphi10$, the flow velocity of the culture medium when the flow rate is 2.2 mL/minute corresponds to the antagonistic suction flow velocity of an aggregate with a particle size of 200 $\mu$m. Therefore, in Comparative Example 1 where the flow rate was 1 mL/minute, the flow velocity of the culture medium was lower than the antagonistic suction flow velocity.

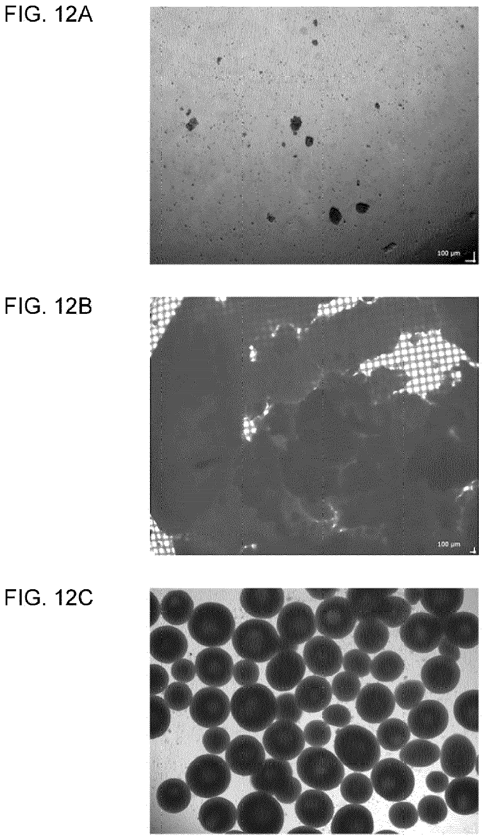

On the fourth day of culture, the growth and proliferation of aggregates in the culture vessel could no longer be seen, so that the culture was stopped, and the cells were observed. FIG. 12A is an optical microscope image of the culture medium in Comparative Example 1. FIG. 12B is an optical microscope image of the filter in Comparative Example 1. As shown in FIG. 12A, in Comparative Example 1, the number of aggregates suspending in the culture medium was small. The cell density in the culture vessel was $9.4 \times 10^4$ cells/mL. Also, as shown in FIG. 12B, many aggregates had adhered to the filter.

In Comparative Example 1, even though the flow velocity of the culture medium was lower than the antagonistic suction flow velocity, the filter was clogged with aggregates. This is because the filter was installed at the tip of the suction tube. That is, it is considered that aggregates that had joined the flow of the culture medium caused by stirring came into contact with the filter and were trapped. Thus, when the filter is exposed in the culture vessel and in contact with the culture medium, even if the flow velocity of the culture medium is lower than the antagonistic suction flow velocity, aggregates may be trapped by the filter, and the cell viability could be significantly reduced.

Example 1

Using the culture device shown in FIG. 1, stirred suspension culture of mNPCs was conducted at an initial cell density of $1 \times 10^5$ cells/mL. The tube diameter of the suction tube was $\varphi4$. From the fifth day of culture when the particle sizes of many aggregates became 400 $\mu$m or larger, suction of the culture medium was started at a flow rate of 1 mL/minute, and the culture was continued while the suctioned culture medium was returned to the culture vessel. As shown in FIG. 8, when the tube diameter of the suction tube is $\varphi4$, the flow velocity of the culture medium when the flow rate is 1.1 mL/minute corresponds to the antagonistic suction flow velocity of an aggregate with a particle size of 400 $\mu$m. Therefore, in Example 1 where the flow rate was 1 mL/minute, the flow velocity of the culture medium was lower than the antagonistic suction flow velocity. In order to prevent suction of aggregates or debris smaller than 400 $\mu$m, a mesh filter with a pore size of 100 um was installed in an upper part within the suction tube.

FIG. 12C is an optical microscope image of the culture medium in Example 1. As shown in FIG. 12C, in Example 1, no decrease in aggregates could be observed compared to Comparative Example 1. The culture was further continued with the target number of days set to 8 days. As a result, it was confirmed that the aggregates could be cultured in good condition for at least 8 days. The cell density in the culture vessel on the eighth day of culture was $4.1 \times 10^6$ cells/mL. Also, the cell viability was 91%. From the above, it can be understood that, with the culture device and culture method according to an embodiment, the culture medium can be suctioned without suctioning the aggregates to be cultured, thereby improving the efficiency of cell culture.

Aggregates removal test

In this test, it was verified that, with the culture device and culture method according to an embodiment, aggregates of specific particle sizes can be removed from aggregates of various particle sizes in the culture medium. Using the culture device shown in FIG. 1, stirred suspension culture of mNPCs was conducted at an initial cell density of $1 \times 10^5$ cells/mL. The culture period was 8 days. In the replacement of the culture medium, after stopping the stirring of the culture medium and confirming that the aggregates had settled, the supernatant was suctioned, and fresh culture medium was added. The culture medium was replaced daily.

After the completion of the stirred suspension culture for 8 days, a suction tube with a tube diameter of φ10 was installed in the culture device. In order to remove aggregates with particle sizes smaller than the target particle size and debris, a mesh filter with a pore size of 100 μm was installed in an upper part within the suction tube. Suction of the culture medium was started at a flow rate of 8.0 mL/minute, and perfusion processing of returning the suctioned culture medium to the culture vessel was continued for 6 hours. As shown in FIG. 8, when the tube diameter of the suction tube is φ10, the flow velocity of the culture medium when the flow rate is 8.0 mL/minute corresponds to the antagonistic suction flow velocity of an aggregate with a particle size of 450 μm. Therefore, aggregates smaller than 450 μm are expected to be suctioned by the suction tube and removed by the mesh filter.

After the completion of the perfusion processing at the flow rate of 8.0 mL/minute for 6 hours, the flow rate was increased to 14.1 mL/minute, and the perfusion processing was further continued for 6 hours. As shown in FIG. 8, when the tube diameter of the suction tube is φ10, the flow velocity of the culture medium when the flow rate is 14.1 mL/minute corresponds to the antagonistic suction flow velocity of an aggregate with a particle size of 650 μm. Therefore, aggregates smaller than 650 μm are expected to be suctioned by the suction tube and removed by the mesh filter.

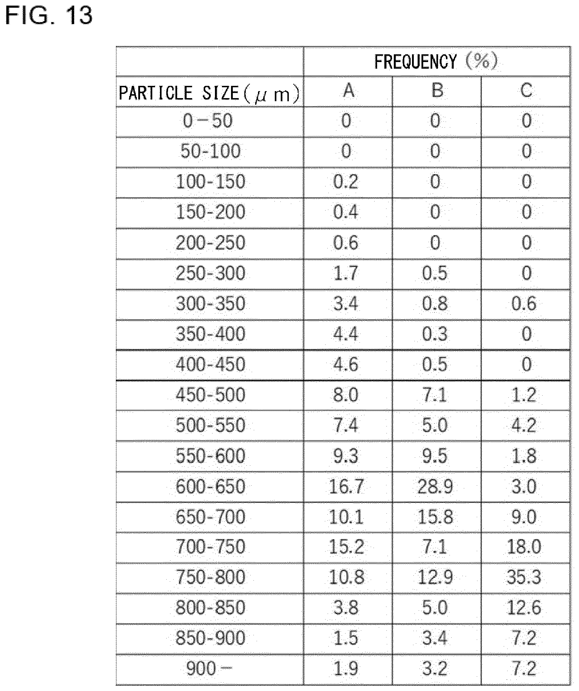

After the stirred suspension culture, after the perfusion processing at the flow rate of 8.0 mL/minute, and after the perfusion processing at the flow rate of 14.1 mL/minute, the culture medium was imaged with an optical microscope (BZ-X500, KEYENCE CORPORATION) to obtain optical microscope images. Then, using measurement software (BZ-X Analyzer, KEYENCE CORPORATION), the particle size of each aggregate in the optical microscope images was measured, and a particle size distribution of the aggregates was obtained. The results are shown in FIG. 13.

FIG. 13 is a diagram that shows an aggregate particle size distribution. The "A" in FIG. 13 is the result after the stirred suspension culture. The "B" in FIG. 13 is the result after the perfusion processing at the flow rate of 8.0 mL/minute. The "C" in FIG. 13 is the result after the perfusion processing at the flow rate of 14.1 mL/minute. As shown in FIG. 13, after the stirred suspension culture, the proportion of aggregates with particle sizes smaller than 450 μm was 15.3%, and the proportion of aggregates with particle sizes of 450 μm or larger was 84.7%. Meanwhile, after the perfusion processing at the flow rate of 8.0 mL/minute, the proportion of aggregates with particle sizes smaller than 450 μm was 2.1%, and the proportion of aggregates with particle sizes of 450 μm or larger was 97.9%.

Also, after the perfusion processing at the flow rate of 8.0 mL/minute, the proportion of aggregates with particle sizes smaller than 650 μm was 52.6%, and the proportion of aggregates with particle sizes of 650 μm or larger was 47.4%. Meanwhile, after the perfusion processing at the flow rate of 14.1 mL/minute, the proportion of aggregates with particle sizes smaller than 650 μm was 10.8%, and the proportion of aggregates with particle sizes of 650 μm or larger was 89.2%. Thus, it was confirmed that, with the culture device and culture method according to an embodiment, aggregates smaller than the target particle size can be suctioned and removed from a group of aggregates of various particle sizes. Therefore, it can be understood that the yield of aggregates having desired particle sizes can be increased.

Aggregate recovery test

In this test, it was verified that, with the culture device and culture method according to an embodiment, aggregates of specific particle sizes can be recovered from aggregates of various particle sizes in the culture medium. Using the culture device shown in FIG. 1, stirred suspension culture of mNPCs was conducted at an initial cell density of $1 \times 10^5$ cells/mL. The culture period was 8 days. In the replacement of the culture medium, after stopping the stirring of the culture medium and confirming that the aggregates had settled, the supernatant was suctioned, and fresh culture medium was added. The culture medium was replaced daily.

After the completion of the stirred suspension culture for 8 days, a suction tube with a tube diameter of φ10 was installed in the culture device. In order to remove aggregates with particle sizes smaller than the target particle size and debris, a mesh filter with a pore size of 100 μm was installed in an upper part within the suction tube. Suction of the culture medium was started at a flow rate of 6.7 mL/minute, and perfusion processing of returning the suctioned culture medium to the culture vessel was continued for 10 hours. As shown in FIG. 8, when the tube diameter of the suction tube is φ10, the flow velocity of the culture medium when the flow rate is 6.7 mL/minute corresponds to the antagonistic suction flow velocity of an aggregate with a particle size of 400 μm. Therefore, aggregates smaller than 400 μm are expected to be suctioned by the suction tube and removed by the mesh filter. When the mesh filter was checked after the perfusion processing, minute aggregates and debris were deposited on the surface of the mesh filter.

After the completion of the perfusion processing at the flow rate of 6.7 mL/minute for 10 hours, a suction tube with a tube diameter of φ10 was newly installed in the culture device. Also, a circulation flow path for returning the culture medium to the culture vessel was connected to the suction tube. Midway along the circulation flow path, a recovery suction tube was provided as a recovery unit for aggregates. The recovery suction tube includes an inlet port and an outlet port on its side surface and has a tube diameter of φ20. Suction of the culture medium was started at a flow rate of 15.7 mL/minute, and perfusion processing of returning the suctioned culture medium to the culture vessel was continued for 12 hours.

As shown in FIG. 8, when the tube diameter of the suction tube is φ10, the flow velocity of the culture medium when the flow rate is 15.7 mL/minute corresponds to the antagonistic suction flow velocity of an aggregate with a particle size of 700 um. Therefore, aggregates smaller than 700 $\mu$m are expected to be suctioned by the suction tube and flow into the recovery unit. Also, the suction tube with a tube diameter of $\varphi$20 has a cross-sectional area four times larger than that of the suction tube with a tube diameter of $\varphi$10. Accordingly, the flow velocity of the culture medium is reduced to 1/4. In this case, the flow velocity of the culture medium is lower than the antagonistic suction flow velocity of an aggregate flowing into the recovery suction tube. Therefore, aggregates of 400 um or larger and smaller than 700 um are expected to settle in a bottom part of the recovery suction tube.

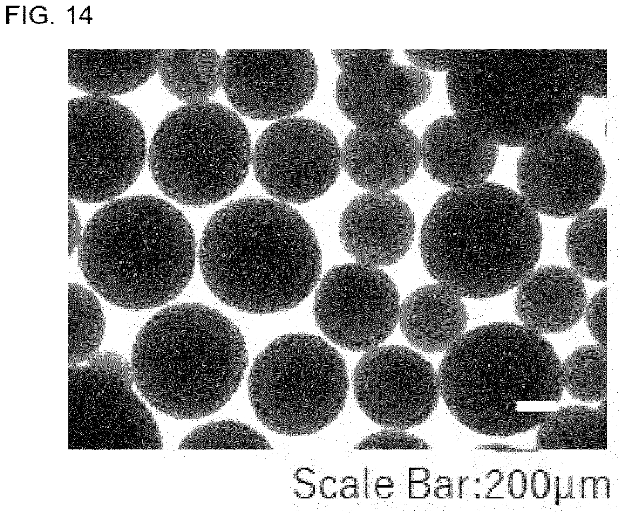

After the completion of the perfusion processing for 12 hours, some of the aggregates were collected from the bottom part of the recovery suction tube into a 24-well plate and imaged with an optical microscope (BZ-X500, KEYENCE CORPORATION) to obtain an optical microscope image. FIG. 14 is an optical microscope image of aggregates deposited in the bottom part of the recovery suction tube. As shown in FIG. 14, it was confirmed that a large number of aggregates having the assumed particle sizes were deposited in the bottom part of the recovery suction tube. Thus, it was confirmed that, with the culture device and culture method according to an embodiment, aggregates having the target particle sizes can be recovered from a group of aggregates of various particle sizes. Therefore, it can be understood that the yield of aggregates having desired particle sizes can be increased.

INDUSTRIAL APPLICABILITY

The present invention is applicable to culture devices and culture methods.

REFERENCE SIGNS LIST

1      culture device
2      culture vessel
4      suction tube
8      suction unit
10     management unit
14     control unit
S      aggregate
W      culture medium

**Claims**

1.  A culture device, comprising:

    a culture vessel that contains one or more aggregates of cells and a culture medium;
    a suction tube that suctions a culture medium in the culture vessel;
    a suction unit that generates suction force in the suction tube; and
    a control unit that controls the suction unit based on an antagonistic suction flow velocity of an aggregate, which is a velocity at which sedimentation of the aggregate is antagonistic to floating of the aggregate due to the suction of the culture medium.

2.  The culture device according to claim 1, wherein the antagonistic suction flow velocity is determined based on a sedimentation velocity prediction curve for the aggregates obtained from: the following Equation (1)
    Math. 1

$$W_* = \frac{w_s}{\sqrt{(s-1)gd}} \qquad (1)$$

where $W_*$ is a dimensionless sedimentation velocity, $w_s$ is a sedimentation velocity of the aggregate [m/s], s is specific gravity given by $\rho_s/\rho_w$, $\rho_s$ is density of the aggregate [kg/m$^3$], $\rho_w$ is density of the culture medium [kg/m$^3$], g is the gravitational acceleration [m/s$^2$], and d is the particle size of the aggregate [m];

the following Equation (2)
Math. 2

$$S_* = \frac{d}{4v}\sqrt{(s-1)gd} \qquad (2)$$

where $S_*$ is a dimensionless particle parameter, $v$ is kinematic viscosity of the culture medium given by $\mu/\rho_w$ [m²/s], $\mu$ is viscosity of the culture medium [Pa·s], $\rho_w$ is density of the culture medium [kg/m³], and d, s, and g are the same as in Equation (1);
the following Equation (3)
Math. 3

$$\frac{1}{W_*} = A + \frac{B}{S_*} \qquad (3)$$

where $W_*$ is the same as in Equation (1), $S_*$ is the same as in Equation (2), and A and B are constants determined from a straight line obtained by plotting, on coordinates in which $1/W_*$ is set on the first axis and $1/S_*$ is set on the second axis, the value for each particle size of the aggregates; and
the following Equation (4)
Math. 4

$$w_s = \frac{(s-1)gd^2}{4vB + Ad\sqrt{(s-1)gd}} \qquad (4)$$

where $w_s$, s, g, and d are the same as in Equation (1), v is the same as in Equation (2), and A and B are the same as in Equation (3).

3. The culture device according to claim 1 or 2, wherein the control unit controls the suction unit so as to suction the culture medium at a flow velocity equal to or lower than the antagonistic suction flow velocity.

4. The culture device according to claim 1 or 2, wherein the control unit controls the suction unit so as to suction the culture medium at a first flow velocity that is higher than the antagonistic suction flow velocity for an aggregate having a predetermined first particle size and that is equal to or lower than the antagonistic suction flow velocity for an aggregate having a second particle size larger than the first particle size.

5. The culture device according to claim 4, wherein the control unit controls the suction unit so as to suction the culture medium at the first flow velocity and then suction the culture medium at a second flow velocity that is higher than the antagonistic suction flow velocity for the aggregate having the second particle size.

6. The culture device according to any one of claims 1 through 5, further comprising a management unit that is connected to the suction tube and that performs at least one of analysis or adjustment of a component of the suctioned culture medium.

7. The culture device according to any one of claims 1 through 6, comprising a plurality of combinations of the culture vessel and the suction tube,

wherein the plurality of combinations are connected with each other,
wherein the tube diameters of the suction tubes are different from each other, and
wherein, when the suction unit is driven, the culture medium is suctioned by the suction tubes and sequentially transferred from the culture vessel on the upstream side to the culture vessel on the downstream side.

8. The culture device according to any one of claims 1 through 6, further comprising a recovery unit for the aggregates including the suction tube,

wherein the suction tube includes an inlet port and an outlet port disposed higher than the inlet port, and the inlet port is connected to the culture vessel, and

wherein, when the suction unit is driven, the culture medium is suctioned by the suction tube to flow into the suction tube through the inlet port and flows up within the suction tube to be discharged through the outlet port, so that, in the suction tube, an aggregate with a particle size corresponding to the tube diameter is recovered.

9.  The culture device according to claim 8,

wherein the recovery unit comprises a plurality of the suction tubes having different tube diameters,

wherein the outlet port of one of adjacent two of the suction tubes is connected to the inlet port of the other of the adjacent two suction tubes, so that the plurality of the suction tubes are connected with each other, and the inlet port of the most upstream suction tube is connected to the culture vessel, and

wherein, when the suction unit is driven, the culture medium is suctioned by the suction tubes and sequentially transferred from the suction tube on the upstream side to the suction tube on the downstream side, so that, in each suction tube, an aggregate with a particle size corresponding to the tube diameter is recovered.

10. A culture method, comprising suctioning, from a culture vessel that contains one or more aggregates of cells and a culture medium, the culture medium at a flow velocity corresponding to an antagonistic suction flow velocity of an aggregate, which is a velocity at which sedimentation of the aggregate is antagonistic to floating of the aggregate due to the suction of the culture medium.

11. The culture method according to claim 10, wherein the antagonistic suction flow velocity is determined based on a sedimentation prediction curve for the aggregates obtained from: the following Equation (1)
Math. 5

$$W_* = \frac{w_s}{\sqrt{(s-1)gd}} \qquad (1)$$

where $W_*$ is a dimensionless sedimentation velocity, $w_s$ is a sedimentation velocity of the aggregate [m/s], s is specific gravity given by $\rho_s/\rho_w$, $\rho_s$ is density of the aggregate [kg/m$^3$], $\rho_w$ is density of the culture medium [kg/m$^3$], g is the gravitational acceleration [m/s$^2$], and d is the particle size of the aggregate [m];

the following Equation (2)
Math. 6

$$S_* = \frac{d}{4v}\sqrt{(s-1)gd} \qquad (2)$$

where $S_*$ is a dimensionless particle parameter, v is kinematic viscosity of the culture medium given by $\mu/\rho_w$ [m$^2$/s], $\mu$ is viscosity of the culture medium [Pa·s], $\rho_w$ is density of the culture medium [kg/m$^3$], and d, s, and g are the same as in Equation (1);
the following Equation (3)
Math. 7

$$\frac{1}{W_*} = A + \frac{B}{S_*} \qquad (3)$$

where $W_*$ is the same as in Equation (1), $S_*$ is the same as in Equation (2), and A and B are constants determined from a straight line obtained by plotting, on coordinates in which $1/W_*$ is set on the first axis and $1/S_*$ is set on the second axis, the value for each particle size of the aggregates; and

EP 4 382 594 A1

the following Equation (4)
Math. 8

$$w_s = \frac{(s-1)gd^2}{4vB + Ad\sqrt{(s-1)gd}} \qquad (4)$$

where $w_s$, s, g, and d are the same as in Equation (1), v is the same as in Equation (2), and A and B are the same as in Equation (3).

FIG. 1

FIG. 2

# FIG. 3

TUBE DIAMETER 6 Φ

| SUCTION FLOW VELOCITY [cm/s] | AGGREGATE PARTICLE SIZE [μm] | | | |
|---|---|---|---|---|
| | 388 | 438 | 598 | 674 |
| 0.1 | A | A | A | A |
| 0.15 | B | A | A | A |
| 0.17 | C | B | A | A |
| 0.24 | C | C | B | A |
| 0.35 | C | C | C | B |

TUBE DIAMETER 8 Φ

| SUCTION FLOW VELOCITY [cm/s] | AGGREGATE PARTICLE SIZE [μm] | | | | |
|---|---|---|---|---|---|
| | 389 | 446 | 574 | 672 | 748.5 |
| 0.1 | A | A | A | A | A |
| 0.13 | B | A | A | A | A |
| 0.21 | C | B | A | A | A |
| 0.24 | C | C | B | A | A |
| 0.37 | C | C | C | B | A |
| 0.39 | C | C | C | C | B |

TUBE DIAMETER 10 Φ

| SUCTION FLOW VELOCITY [cm/s] | AGGREGATE PARTICLE SIZE [μm] | | | | |
|---|---|---|---|---|---|
| | 388 | 469 | 577 | 679 | 739 |
| 0.1 | A | A | A | A | A |
| 0.12 | B | A | A | A | A |
| 0.19 | C | B | A | A | A |
| 0.21 | C | C | B | A | A |
| 0.32 | C | C | C | B | A |
| 0.34 | C | C | C | C | B |

FIG. 4

FIG. 5

## FIG. 6

SEDIMENTATION VELOCITY PREDICTION CURVE

## FIG. 7

ANTAGONISTIC SUCTION FLOW VELOCITY PREDICTION CURVE

# FIG. 8

| AGGREGATE PARTICLE SIZE [μm] | SEDIMENTATION VELOCITY PREDICTED VALUE [cm/s] | ANTAGONISTIC SUCTION FLOW VELOCITY PREDICTED VALUE [cm/s] | ANTAGONISTIC FLOW VELOCITY WHEN Φ4 IS USED [mL/min] | ANTAGONISTIC FLOW VELOCITY WHEN Φ10 IS USED [mL/min] | ANTAGONISTIC FLOW VELOCITY WHEN Φ50 IS USED [mL/min] |
|---|---|---|---|---|---|
| 100 | 0.02 | 0.02 | 0.1 | 0.9 | 23 |
| 150 | 0.03 | 0.03 | 0.2 | 1.5 | 37 |
| 200 | 0.06 | 0.05 | 0.4 | 2.2 | 56 |
| 250 | 0.09 | 0.07 | 0.5 | 3.1 | 78 |
| 300 | 0.12 | 0.09 | 0.7 | 4.2 | 105 |
| 350 | 0.16 | 0.11 | 0.9 | 5.4 | 134 |
| 400 | 0.20 | 0.14 | 1.1 | 6.7 | 166 |
| 450 | 0.25 | 0.17 | 1.3 | 8.0 | 201 |
| 500 | 0.29 | 0.20 | 1.5 | 9.5 | 237 |
| 550 | 0.34 | 0.23 | 1.8 | 11.0 | 275 |
| 600 | 0.39 | 0.27 | 2.0 | 12.5 | 313 |
| 650 | 0.45 | 0.30 | 2.3 | 14.1 | 353 |
| 700 | 0.50 | 0.33 | 2.5 | 15.7 | 393 |
| 750 | 0.55 | 0.37 | 2.8 | 17.4 | 434 |
| 800 | 0.60 | 0.40 | 3.0 | 19.0 | 475 |
| 850 | 0.66 | 0.44 | 3.3 | 20.6 | 516 |
| 900 | 0.71 | 0.47 | 3.6 | 22.3 | 557 |
| 650 | 0.77 | 0.51 | 3.8 | 23.9 | 598 |
| 1000 | 0.82 | 0.54 | 4.1 | 25.6 | 639 |
| 1100 | 0.92 | 0.61 | 4.6 | 28.8 | 720 |
| 1200 | 1.03 | 0.68 | 5.1 | 31.9 | 799 |
| 1300 | 1.13 | 0.74 | 5.6 | 35.0 | 876 |
| 1400 | 1.23 | 0.81 | 6.1 | 38.1 | 951 |
| 1500 | 1.32 | 0.87 | 6.6 | 41.0 | 1025 |

FIG. 9

FIG. 10

EP 4 382 594 A1

FIG. 11

FIG. 12A

FIG. 12B

FIG. 12C

FIG. 13

| PARTICLE SIZE (μm) | FREQUENCY (%) | | |
|---|---|---|---|
| | A | B | C |
| 0-50 | 0 | 0 | 0 |
| 50-100 | 0 | 0 | 0 |
| 100-150 | 0.2 | 0 | 0 |
| 150-200 | 0.4 | 0 | 0 |
| 200-250 | 0.6 | 0 | 0 |
| 250-300 | 1.7 | 0.5 | 0 |
| 300-350 | 3.4 | 0.8 | 0.6 |
| 350-400 | 4.4 | 0.3 | 0 |
| 400-450 | 4.6 | 0.5 | 0 |
| 450-500 | 8.0 | 7.1 | 1.2 |
| 500-550 | 7.4 | 5.0 | 4.2 |
| 550-600 | 9.3 | 9.5 | 1.8 |
| 600-650 | 16.7 | 28.9 | 3.0 |
| 650-700 | 10.1 | 15.8 | 9.0 |
| 700-750 | 15.2 | 7.1 | 18.0 |
| 750-800 | 10.8 | 12.9 | 35.3 |
| 800-850 | 3.8 | 5.0 | 12.6 |
| 850-900 | 1.5 | 3.4 | 7.2 |
| 900- | 1.9 | 3.2 | 7.2 |

FIG. 14

Scale Bar:200μm

## INTERNATIONAL SEARCH REPORT

| International application No. |
|---|
| **PCT/JP2022/028831** |

**A.    CLASSIFICATION OF SUBJECT MATTER**

*C12M 1/00*(2006.01)i; *C12M 3/00*(2006.01)i
FI:    C12M1/00 D; C12M1/00 Z; C12M3/00

According to International Patent Classification (IPC) or to both national classification and IPC

**B.    FIELDS SEARCHED**

Minimum documentation searched (classification system followed by classification symbols)

C12M1/00; C12M3/00

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C.    DOCUMENTS CONSIDERED TO BE RELEVANT**

| Category* | Citation of document, with indication, where appropriate, of the relevant passages | Relevant to claim No. |
|---|---|---|
| X | WO 2020/032041 A1 (NISSAN CHEMICAL CORP) 13 February 2020 (2020-02-13) claims, paragraphs [0001]-[0090] | 1-11 |
| A | WO 2016/020989 A1 (YAMAHA MOTOR CO LTD) 11 February 2016 (2016-02-11) claim, paragraphs [0001]-[0040] | 1-11 |
| A | WO 2019/124540 A1 (UNIV KYOTO) 27 June 2019 (2019-06-27) claims, examples | 1-11 |
| A | WO 2020/008606 A1 (MITSUBISHI ELECTRIC CORP et al.) 09 January 2020 (2020-01-09) claims | 1-11 |
| A | WO 2015/122528 A1 (ASAHI KASEI KABUSHIKI KAISHA et al.) 20 August 2015 (2015-08-20) claims | 1-11 |

☐ Further documents are listed in the continuation of Box C.          ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **07 October 2022** | **18 October 2022** |

| Name and mailing address of the ISA/JP | Authorized officer |
|---|---|
| **Japan Patent Office (ISA/JP)** <br> **3-4-3 Kasumigaseki, Chiyoda-ku, Tokyo 100-8915** <br> **Japan** | |
| | Telephone No. |

Form PCT/ISA/210 (second sheet) (January 2015)

**INTERNATIONAL SEARCH REPORT**
Information on patent family members

International application No.

**PCT/JP2022/028831**

| Patent document cited in search report | | | Publication date (day/month/year) | Patent family member(s) | | | Publication date (day/month/year) |
|---|---|---|---|---|---|---|---|
| WO | 2020/032041 | A1 | 13 February 2020 | US 2021/0317394 A1 claims, paragraphs [0001]-[0182] EP 3831926 A1 | | | |
| WO | 2016/020989 | A1 | 11 February 2016 | (Family: none) | | | |
| WO | 2019/124540 | A1 | 27 June 2019 | US 2020/0392445 A1 claims, examples EP 3730597 A1 | | | |
| WO | 2020/008606 | A1 | 09 January 2020 | US 2021/0054330 A1 claims | | | |
| WO | 2015/122528 | A1 | 20 August 2015 | US 2016/0355774 A1 claims EP 3109314 A1 | | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2019124540 A

**Non-patent literature cited in the description**

- **JIMENEZ, J. A. ; MADSEN, O. S.** A simple formula to estimate settling velocity of natural sediments. *Journal of Waterway, Port, Coastal, and Ocean Engineering,* 2003, vol. 129 (2), 70-78

- *Measurement,* vol. 9 (10